# EUROPÄISCHE PATENTSCHRIFT

(11) **EP 0 598 881 B1**
(45) Veröffentlichungstag und Bekanntmachung des Hinweises auf die Patenterteilung: **15.09.1999**
(21) Anmeldenummer: 93912960.7
(22) Anmeldetag: 12.06.1993
(51) Int. Cl.: A61M 29/04, A61B 17/00, A61L 31/00

(54) **SELBSTTÄTIG EXPANDIERENDER GEWEBEEXPANDER**
SELF-INFLATING TISSUE EXPANDER
DILATATEUR TISSULAIRE AUTODILATABLE

(30) Priorität: 12.06.1992 DE 4219207
(43) Veröffentlichungstag der Anmeldung: 01.06.1994
(73) Patentinhaber: WIESE, Günter K., 37075 Göttingen (DE)
(72) Erfinder: WIESE, Günter K., 37075 Göttingen (DE)
(74) Vertreter: Rehberg, Elmar, Dipl.-Ing.
(86) Internationale Anmeldenummer: EP9301490
(87) Internationale Veröffentlichungsnummer: WO9325266

(56) Entgegenhaltungen:
- US-A- 3 867 329
- US-A- 4 237 893
- US-A- 5 015 238
- US-A- 5 074 878
- PLASTIC AND RECONSTRUCTIVE SURGERY, vol. 70, no. 1-6, 1982, E.D. AUSTADT et al. "A Self-Inflating Tissue Expander", Seiten 588-594

## Beschreibung

Die Erfindung bezieht sich auf einen selbsttätig expandierenden Gewebeexpander nach dem Oberbegriff des Anspruchs 1. Um Hohlräume für eine Einfügung von Implantaten zu schaffen, aber auch um gesundes Gewebe für eine Eigentransplantation bereitzustellen, bedient man sich der Methode der kontrollierten Gewebeexpansion. Hierbei wird das Gewebe unter mäßiger Druckanwendung kontinuierlich geweitet, bis der gewünschte Hohlraum bzw. die gewünschte Menge zusätzlichen Gewebes vorliegt.

Aus der Dissertation "Controlled Tissue-Expansion in Reconstructive Surgery" (Julian H.A. van Rappard, Thesis Groningen, Die Niederlande, 1988) ist ein Gewebeexpander bekannt, der durch schrittweise Füllung mit einer Flüssigkeit expandiert wird. Der Gewebeexpander weist eine undurchlässige, dehnfeste Haut und ein selbstabdichtendes Ventil zum Einfüllen von Flüssigkeit mit Hilfe einer Hohlnadel und einer Spritze auf. Der Gewebeexpander wird unter das zu expandierende Gewebe implantiert, wobei das Ventil so anzuordnen ist, daß es von außen mit der Hohlnadel erreicht werden kann. Zur eigentlichen Expansion des Gewebes wird der Gewebeexpander schrittweise mit Flüssigkeit gefüllt. Hierzu wird die Hohlnadel jeweils durch das Gewebe in das Ventil gestochen und dann die Flüssigkeit mit der Spritze in den Gewebeexpander eingespritzt. Der Gewebeexpander erreicht mit seiner vollständigen Füllung durch die Flüssigkeit eine durch die Gestaltung seiner Haut vorgegebene Form. Die Gestaltung der Haut des Gewebeexpanders ist so an verschiedene Aufgaben anpaßbar. Vorteilhaft ist bei dem bekannten Gewebeexpander, daß er eine genau kontrollierte Expansion des Gewebes ermöglicht. Als äußerst nachteilig erweist sich jedoch das Auftreten von hohen Spitzendrücken nach jedem Einfüllen von Flüssigkeit in den Gewebeexpander. Hiervon sind insbesondere die Bereiche des zu expandierenden Gewebes betroffen, die direkt an dem Gewebeexpander angrenzen. Diese Bereiche werden sogar so stark komprimiert, daß Gewebeschäden auftreten. Bei Reduktion der Flüssigkeitsmenge, die in jedem Einzelschritt in den Gewebeexpander eingebracht wird, resultieren Probleme aus dem häufigen Durchstechen des Gewebes im Bereich des Ventils. Außerdem kann das Ventil undicht werden, wodurch der Gewebeexpander seine Funktion einbüßt. Gefahren für das den Gewebeexpander umgebende Gewebe resultieren aus dem Undichtwerden des Ventils nicht, solange eine physiologisch unbedenkliche, sterile Flüssigkeit zum Befüllen des Gewebeexpanders verwendet wird.

Ein selbsttätig expandierender Gewebeexpander der eingangs beschriebenen Art ist aus dem Artikel "A Self-Inflating Tissue Expander" (E.D. Austad et al., Plastic and reconstructive surgery, Vol. 70, No. 5, Seiten 588 ff.) bekannt. Dieser Gewebeexpander besteht aus einer mit einer Kochsalzlösung gefüllten Silikonmembran. Die Molarität der Kochsalzlösung liegt oberhalb der physiologischen Molarität von etwa 0,3. Hierauf beruht eine osmotische Triebkraft, die Körperflüssigkeit aus dem den Gewebeexpander umgebenden Gewebe durch die semipermeabel ausgebildete Silikonmembran in den Gewebeexpander hineintreibt. Die Expansion des Gewebeexpanders und damit auch des den Gewebeexpander umgebenden Gewebes erfolgt so, ohne daß eine Manipulation von außen erforderlich ist. Darüberhinaus weist das den Gewebeexpander umgebende Gewebe nach der Expansion eine hervorragende, unbeschädigte Qualität auf. Dies ist einerseits darauf zurückzuführen, daß der selbsttätig expandierende Gewebeexpander keine Druckspitzen aufbaut und daß andererseits die Aufnahme von Körperflüssigkeit in den Expander den Stoffwechsel des ihn umgebenden Gewebes fördert. Als nachteilig erweist sich demgegenüber die geringe Volumenvergrößerung des Gewebeexpanders, sofern die Molarität der Kochsalzlösung am Anfang ein physiologisch vertretbares Maß nicht bei weitem übersteigt. Außerdem macht sich negativ bemerkbar, daß sich die Eigenschaften der Silikonmembran mit ihrer Dehnung stark verändern. Insbesondere wächst die Porengröße der Silikonmembran stetig an. So können mit anwachsender Dehnung auch Kochsalzionen in anwachsendem Umfang durch die Silikonmembran hindurchtreten. Dies führt jedoch zu einem Abbau der osmotischen Triebkraft, ohne daß hierdurch ein Volumengewinn für den Gewebeexpander verbunden wäre. Ein weiterer Nachteil des bekannten Gewebeexpanders ist die fehlende Möglichkeit, die erfolgende Expansion des Gewebes in ihrer Richtung zu steuern. Die Gestaltung der Silikonmembran hat nur einen geringen Einfluß auf die Form des Gewebeexpanders nach seiner Expansion. Darüberhinaus ist festzustellen, daß die Expansion der Silikonmembran selbst einen beachtlichen Teil der osmotischen Triebkraft bei dem Gewebeexpander aufbraucht. Hinzu kommt, daß die Dehnung der Silikonmembran mit zunehmendem Volumen des Gewebeexpanders immer mehr Kraft erfordert, während die osmotische Triebkraft gleichzeitig zurückgeht. So steht für die Expansion des den Gewebeexpander umgebenden Gewebes nur noch eine stark abnehmende resultierende Triebkraft zur Verfügung und das Verhältnis von der Ausgangsgröße zu der erreichbaren Endgröße des Gewebeexpanders wird über das rein rechnerische Maß weiter begrenzt.

Aus der US-Patentschrift 4 237 893 ist eine Vorrichtung zum Aufweiten des Zervikalkanals (Muttermund) bekannt. Die Vorrichtung weist stabförmige äußere Abmessungen und einen mindestens dreischichtigen inneren Aufbau auf. Eine Zwischenschicht ist dabei aus hydrophilem Polymermaterial, d. h. aus einem Hydrogel, ausgebildet. Die Vorrichtung wird in den Zervikalkanal eingeführt und weitet sich dort durch Aufnahme von Körperflüssigkeit aus der Gebärmutter auf. Dabei wird der Zervikalkanal in seinem Querschnitt gedehnt. Wenn nach wenigen Stunden die gewünschte Öffnung des Zervikalkanals erreicht ist, wird die Vorrichtung aus dem Zervikalkanal entnommen und ein chirurgischer Eingriff kann durch den Zervikalkanal erfolgen. Mit der bekannten Vorrichtung wird zwar eine bestehende Körperhöhle temporär geweitet, es wird jedoch weder eine neue Körperhöhlung geschaffen, noch zusätzliches Gewebe gebildet. Darüberhinaus ist die bekannte Vorrichtung zum Aufweiten des Zervikalkanals nicht zum Implantieren in ein Gewebe, sondern zum Einführen in eine bereits vorhandene, offene Körperhöhle bestimmt.

Die US-Patentschrift 3 867 329 beschreibt ein Verfahren zur Herstellung eines stabförmigen Formkörpers aus Hydrogel, der als Vorrichtung zum Aufweiten des Zervikalkanals dienen soll. Dabei wird zunächst eine Copolymerisation verschiedener wässriger Substanzen durchgeführt und das entstehende Copolymer anschließend aufbereitet. Die resultierenden Hydrogele weisen in destilliertem Wasser einen Schwellungskoeffizienten von bis zu 25 nach fünf Tagen auf. Angaben zu einem Schwellungskoeffizienten in physiologischer Kochsalzlösung enthält die US-Patentschrift nicht.

Aus der US-Patentschrift 3 975 350 ist es bekannt, ein Hydrogel aus einem Polyurethanpolymer als implantierfähigen Medikamententräger zu verwenden. Der Aspekt einer Gewebedehnung wird in der US-Patentschrift nicht angesprochen.

Es ist bekannt, sogenannte weiche Kontaktlinsen aus Hydrogel herzustellen. Unter dem Oberbegriff Hydrogel versteht man polymere Substanzen, die sich in einer wässrigen Umgebung unter Aufnahme von Wasser ausdehnen. Der Umfang der Ausdehnung ist je nach Hydrogel sehr unterschiedlich. Er wird quantitativ als Schwellungskoeffizient angegeben. Dabei bedeutet ein Schwellungskoeffizient von n, daß sich das Ausgangsvolumen unter Aufnahme von Wasser auf das n-fache vergrößert hat. Bestandteil des Schwellungskoeffizienten ist die Angabe der Lösung, in der er bestimmt wurde. So ist unmittelbar nachvollziehbar, daß sich in destilliertem Wasser aufgrund des höheren osmotischen Gefälles immer ein größerer Schwellungskoeffizient ergibt als beispielsweise in physiologischer Kochsalzlösung. Das Hydrogel, aus dem weiche Kontaktlinsen hergestellt werden, weist in physiologischer Kochsalzlösung einen Schwellungskoeffizienten von weniger als 4 auf. Daneben zeigt es eine vorteilhaft große Formstabilität und Reißfestigkeit in geguollenem Zustand.

Die weichen Kontaktlinsen Geaflex 70 der Firma wöhlk-contact-linsen bestehen aus einem Copolymerisat aus Methylmethacrylat (MMA) und Vinylpyrrolidon (VP). Hierbei handelt es sich um, ein lösungsmittelfrei vernetzes, nicht ionisches Copolymer mit freien Methylenseitenketten.

Der Erfindung liegt die Aufgabe zugrunde, einen selbsttätig expandierenden Gewebeexpander aufzuzeigen, der eine insbesondere in ihrer Richtung kontrollierte Expansion des ihn umgebenden Gewebes auch in größerem Umfang ermöglicht.

Erfindungsgemäß wird dies durch die Merkmale des Anspruchs 1 erreicht. Der Gewebeexpander besteht ausschl. aus einem Formkörper aus Hydrogel. Es versteht sich, daß hierbei nur ein Hydrogel in Frage kommt, das seine Ausgangsform beibehält oder zumindest durch seine Wasseraufnahme nicht aufgelöst wird. Anderenfalls wäre seine Entfernung nach der erfolgten Expansion des Gewebes problematisch. Ein Hydrogel, welches für die Herstellung des Formkörpers geeignet ist, ist das Hydrogel, aus dem die bekannten, weichen Kontaktlinsen gefertigt werden.

Ein noch verbessertes Quellvermögen wird erreicht, wenn das Hydrogel ein ionisches Hydrogel ist. Die Osmolarität des Hydrogels wird dabei durch die Ionen-Anionen-Dissoziation des Hydrogels in wässriger Lösung gesteigert.

Besonders gut als Bestandteile von Gewebeexpandern sind ionische Hydrogele geeignet, die auf der Basis eines fertig vernetzen, nichtionischen Polymers ausgebildet werden. Auf diese Weise weist das ionische Hydrogel die mechanische Stabilität eines nichtionischen Polymers, aber gleichzeitig einen gegenüber nichtionischen Hydrogelen deutlich gesteigerten Schwellungskoeffizienten auf. Auf vergleichsweise einfachem Wege ist ein mechanisch stabiles ionisches Hydrogel erreichbar, wenn ein nichtionisches Hydrogel verseift wird.

Das verseifbare nichtionische Hydrogel kann ein Polymer auf der Basis von Methylmethacrylat (MMA) sein. Derartige Polymere weisen Methylenseitenketten auf, die beispielsweise durch Einwirkung von Natronlauge und unter Abspaltung von Methylen in Carboxylseitengruppen umgewandelt werden. In wässriger Lösung dissoziieren die Carboxylgruppen zu negativ geladenen CO₂⁻-Gruppen und freien H⁺-Ionen.

Der Formkörper aus Hydrogel kann in mehrere Einzelkörper aufgeteilt sein. Der Zeitraum, in dem das Hydrogel seine maximale Ausdehnung erreicht, wird im wesentlichen durch die Abmessungen des Formkörpers bestimmt. So läßt sich die Ausdehngeschwindigkeit des Formkörpers durch seine Aufteilung in Einzelkörper beschleunigen. Hierbei wird einerseits der von dem Wasser in dem Hydrogel maximal zurückzulegende Weg begrenzt und andererseits eine größere Oberfläche für den Eintritt des Wassers in das Hydrogel bereitgestellt.

In dem Gewebeexpander kann ein gasgefüllter Druckpuffer vorgesehen sein. Gasgefüllte Druckpuffer sind hervorragend zum Abfangen von Druckspitzen geeignet. Bei dem neuen Gewebeexpander treten im Gegensatz zu nicht selbst expandierenden Gewebeexpandern zwar keine eigentlichen Druckspitzen auf, der von dem Gewebeexpander auf das ihn umgebende Gewebe ausgeübte Druck kann durch die Einfügung des Druckpuffers jedoch noch gleichmäßiger gestaltet werden. Als Füllgas für den Druckpuffer ist insbesondere Kohlendioxid geeignet, da dieses bei Freiwerden durch das den Gewebeexpander umgebende Gewebe resorbierbar ist. Der Druckpuffer kann an beliebiger Stelle innerhalb des Gewebeexpanders angeordnet sein, so beispielsweise innerhalb des Formkörpers aus Hydrogel.

Der Formkörper bzw. die Einzelkörper aus Hydrogel können teilweise mit einem Metall, insbesondere einem Edelmetall bedampft sein. Durch die teilweise Bedampfung mit Metall wird die aktive Oberfläche des Formkörpers bzw. der Einzelkörper verringert. Hierdurch reduziert sich auch ihre Quellgeschwindigkeit. Dies ist sinnvoll, wenn beispielsweise eine besonders langsame Expansion von Gewebe durch den Gewebeexpander erreicht werden soll.

Die Erfindung wird im Folgenden anhand von Ausführungsbeispielen näher erläutert und beschrieben. Es zeigt:
- Figur 1: eine Ausführungsform des selbsttägig expandierenden Gewebeexpanders,
- Figur 2: eine Strukturformel zu dem Gewebeexpander gemäß Figur 1 und
- Figur 3: eine Strukturformel zu einer weiteren Ausführungsform des Gewebeexpanders.

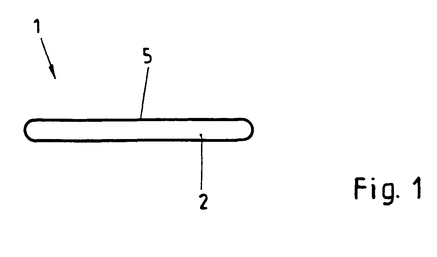

Der in Figur 1 dargestellte etwa stabförmige Gewebeexpander 1 ist für die Expansion von Knochenhaut vorgesehen. Genauer gesagt dient er dazu, die Knochenhaut auf der Oberseite eines Kieferkamms aufzuweiten, bis in die entstehende Knochenhauttasche genügend knochenbildendes Material zum Aufbau eines erhöhten Kieferkamms eingebracht werden kann. Die Erhöhung des Kieferkamms ist vielfach Voraussetzung für den sinnvollen Einsatz von Zahnprothesen bei Patienten, die bereits über längere Zeit zahnlos sind. Der Gewebeexpander 1 besteht hier einzig und allein aus einem Formkörper 5 aus einem Hydrogel 2. Das Hydrogel ist in trockenem Zustand, vor seiner Implantation starr, so daß es problemlos von einer Schmalseite in eine Tasche zwischen dem Kieferkamm und der angehobenen Knochenhaut eingeschoben werden kann. Das Hydrogel (2) basiert auf einem Copolymerisat von Methylmethacrylat (MMA) und Vinylpyrrolidon (VP). Weiterhin enthält das Hydrogel 2 Zusätze, mit denen eine gute mechanische und Formstabilität des Gewebeexpanders 1 erreicht wird. Das Hydrogel 2 ist in seiner Zusammensetzung identisch mit demjenigen, das zur Herstellung der weichen Kontaktlinsen "Geaflex 70" der Firma "wöhlk-contact-linsen" verwendet wird. Die physiologische Verträglichkeit dieses Hydrogels ist bereits weitgehend nachgewiesen. In physiologischer Kochsalzlösung quillt das Hydrogel unter Aufnahme von Wasser, bis es sein 3,6-faches Ausgangsvolumen erreicht hat. Hierbei werden ca. 220 % des Ausgangsgewichts des Hydrogel an Wasser aufgenommen. Die Triebkraft dieser Wasseraufnahme ist osmotischer Natur, wobei die Oberfläche des Hydrogels die Funktion einer Membran übernimmt. Einen ähnlichen Quellungsgrad wie das Hydrogel 2 in physiologischer Kochsalzlösung erreicht der Gewebeexpander 1 innerhalb eines ihn umgebenden, menschlichen Gewebes, da sämtliche Körperflüssigkeit des Menschen mit physiologischer Kochsalzlösung recht genau in osmotischem Gleichgewicht steht. Abschläge sind aber zur Berücksichtigung der Widerstandskraft zu machen, die das zu expandierende Gewebe, im folgenden Fall also die Knochenhaut, seiner Dehnung durch den Gewebeexpander 1 entgegensetzt. In dem Fall der Knochenhautexpansion ist der im Gewebe erreichbare Quellungsgrad von etwa dem 3-fachen des Ausgangsvolumens jedoch ausreichend. Als besonders vorteilhaft erweist sich dabei, daß der Formkörper 5 des Gewebeexpanders 1 auch in gequollenem Zustand ein einzelnes, reißfest zusammenhängendes Stück Hydrogel bleibt und keine Auflösungserscheinungen zeigt. Dies erleichtert in ganz besonderem Maße die Explantation des Gewebeexpanders 1. Nach der Explantation ist festzustellen, daß die von dem Gewebeexpander ausgedehnte Knochenhaut eine hervorragende Konstitution aufweist, da sie allein durch die Wasseraufnahme des Gewebeexpanders einem stetigen Stoffwechsel ausgesetzt war. Die unter die Knochenhaut zur Aufstockung des Kieferkamms eingebrachten knochenbildenden Materialien werden so rasch zu fester Knochensubstanz umgesetzt.

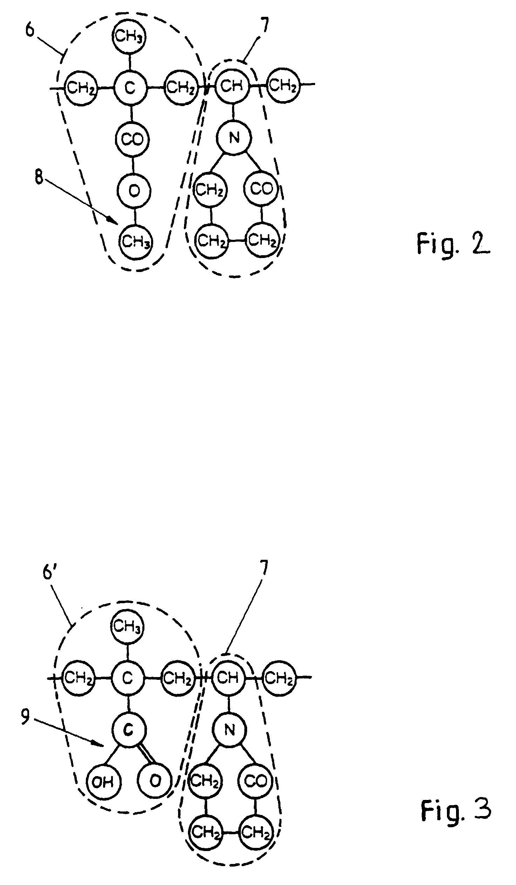

Es gibt eine Möglichkeit, den Schwellungskoeffizienten des Gewebeexpanders aus Hydrogel gemäß Figur 1 zu steigern. Diese Möglichkeit sei anhand der Figuren 2 und 3 erläutert, wobei Figur 2 die Strukturformel des Hydrogels 2 des Formkörpers 5 gemäß Figur 1 und Figur 3 die Strukturformel eines weiteren, hier nicht separat dargestellten Formkörpers einer weiteren Ausführungsform des Gewebeexpanders wiedergibt. Wie bereits ausgeführt ist das Hydrogel 2 des Formkörpers 5 gemäß Figur 1 ein Copolymer aus Methylenmethacrylat (MMA) 6 und Vinylpyrrolidon (VP) 7. Dabei weist die Struktur wie in Figur 2 wiedergegeben freie Methylenseitenketten 8 auf. Bei einem Verseifen der Struktur gemäß Figur 2 mit Natronlauge entsteht unter Abspaltung von Methylen die Struktur gemäß Figur 3. Hier liegt statt der Methylgruppe 8 eine freie Carboxylgruppe 9 im Bereich der Methacrylgruppe 6' vor. In wässriger Lösung dissoziiert die Carboxylgruppe in einen positiv geladenen Rest CO₂⁻ und ein freies Ion H⁺. Auf diese Weise wird die Osmolarität des Hydrogels 2 durch die Verseifung erhöht. Ein Hydrogel 2 mit der Strukturformel gemäß Figur 3 weist einen Schwellungskoeffizienten in destilliertem Wasser von mehr als 30 und in physiologischer Kochsalzlösung von etwa 10 bis 12 auf. Dennoch ist die mechanische Stabilität des Hydrogels selbst nach der Sättigung mit Wasser noch als gut zu bezeichnen. Dies ist darauf zurückzuführen, daß die für die mechanischen Eigenschaften wesentliche Grundstruktur des Polymers durch die Verseifung nicht verändert wurde.

Im Folgenden wird ein Verfahren beschrieben, mit dem das Copolymerisat von Methylmethacrylat (MMA) und Vinylpyrrolidon (VP) des Gewebeexpanders gemäß Figur 1 erfolgreich behandelt wurde, um seine Schwellungseigenschaften erheblich zu verbessern. Die folgenden Zahlenangaben beziehen sich dabei auf etwa 1 cm³ große, kompakte Polymerabschnitte. Für größere Polymerabschnitte sind die Zeitangaben wegen der längeren Diffusionszeiten zu erhöhen, für kleinere oder solche mit besonders großer relativer Oberfläche herabzusetzen. Zunächst wird das Copolymerisat für eine Dauer von fünf Tagen mit einmolarer Natronlauge verseift. Anschließend wird es für 30 Tage in destilliertem Wasser, das mehrfach gewechselt wird, gewaschen, um Reste von Natronlauge aus dem Copolymerisat zu entfernen. Bereits nach der Verseifung weist das Copolymerisat die in Figur 3 wiedergegebene Struktur auf. Nach dem Waschen wird das Copolymerisat in Natriumchloridlösungen mit aufsteigender Konzentration equilibriert. Hierbei setzt ein osmotisches Schrumpfen des zuvor mit destilliertem Wasser gesättigten Copolymerisats ein. Geeignet sind Konzentrationen der Natriumchloridlösung beginnend mit 0,1 %, über 0,3 und 0,5 % aufsteigend auf 0,9 %. Das Equilibrieren erfolgt in der jeweiligen Lösung über einen Zeitraum von 1 bis 3 Tagen. Der Endwert der Konzentration der Natriumchloridlösung von 0,9 % entspricht demjenigen physiologischer Kochsalzlösung. Das noch nicht vollständig entwässerte Copolymerisat wird in eine keimdichte, aber wasserdampfdurchlässige Verpackung eingebracht und darin für 10 Minuten bei 120 ° in einem Autoklaven sterilisiert. Abschließend erfolgt eine Auslagerung bei Zimmertemperatur und reduzierter Luftfeuchtigkeit, um den Wassergehalt des Copolymerisats soweit zu reduzieren, daß es nahezu wasserfrei ist. Das aus der keimdichten Verpackung entnommene Copolymerisat weist in physiologischer Kochsalzlösung einen Schwellungskoeffizienten von 12 auf.

In einer Abwandlung des vorgeschriebenen Verfahrens wird das Copolymerisat zusätzlich in einer Natriumchloridlösung equilibriert, deren Konzentration überphysiologisch ist und beispielsweise eine Konzentration von 1,2 % aufweist.

Hierdurch wird in größerem Umfang eine Absättigung der durch Dissoziation ionisierten Carboxylgruppen 9 des Copolymerisats mit Hilfe der in der Kochsalzlösung dissoziiert vorliegenden NA⁺-Ionen erreicht. Hieraus resultieren quasi-nichtionische Eigenschaften des entwässerten Hydrogels bei der erneuten Aufnahme von Wasser, bis die Na⁺-Ionen aus dem Copolymerisat herausdiffundiert sind. Die damit einhergehende anfangs verringerte Schwellungsgeschwindigkeit des Gewebeexpanders ist als vorteilhaft anzusehen, da sie insbesondere eine übermäßige Belastung des den Gewebeexpander umgebenden Gewebes nach dem Implantieren verhindert.

## Patentansprüche

1. Selbstätig expandierender Gewebeexpander zur Schaffung von Hohlräumen für eine Einfügung von Implantaten oder zur Bereitstellung von Gewebe für eine Eigentransplantation, wobei der Gewebeexpander seinerseits in das Gewebe implantiert wird und dort aufgrund einer osmotischen Triebkraft Körperflüssigkeit, insbesondere Wasser, aus dem ihn umgebenden Gewebe aufnimmt, **dadurch gekennzeichnet**, daß der Gewebeexpander aus einem Formkörper (5) aus einem vernetzten, physiologisch verträglichen Hydrogel (2) besteht.

2. Gewebeexpander nach Anspruch 1, **dadurch gekennzeichnet**, daß das Hydrogel (2) ein ionische Hydrogel ist.

3. Gewebeexpander nach Anspruch 2, **dadurch gekennzeichnet**, daß das ionische Hydrogel ein verseiftes nichtionisches Hydrogel ist.

4. Gewebeexpander nach Anspruch 3, **dadurch gekennzeichnet**, daß das nichtionische Hydrogel ein Polymer auf der Basis von Methylmethacrylat (MMA) ist.

## Claims

1. Self-inflating tissue expander to create cavities for the insertion of implants or to provide tissue for an own transplantation, the tissue expander itself being implanted into the tissue, where it absorbs body fluid, especially water, from the surrounding tissue due to an osmotic driving force, **characterized in that** the tissue expander is a shaped body (5) of a cross-linked, physiologically acceptable hydrogel (2).

2. Tissue expander according to claim 1, **characterized in that** the hydrogel (2) is an ionic hydrogel.

3. Tissue expander according to claim 2, **characterized in that** the ionic hydrogel is a saponified non-ionic hydrogel.

4. Tissue expander according to claim 3, **characterized in that** the non-ionic hydrogel is a polymer on the basis of methylmethacrylate (MMA).

## Revendications

1. Dilatateur tissulaire autodilatable, pour la réalisation de cavités pour l'insertion d'implants ou pour la préparation de tissus pour une auto-transplantation, le dilatateur tissulaire étant implanté de son côté dans le tissu et y absorbant du liquide corporel, en particulier de l'eau, du tissu qui l'entoure en raison d'une force de propulsion osmotique, caractérisé en ce que le dilatateur tissulaire est constitué d'un corps façonné (5) fait d'un hydrogel (2) réticulé, toléré physiologiquement.

2. Dilatateur tissulaire selon la revendication 1, caractérisé en ce que l'hydrogel (2) est un hydrogel ionique.

3. Dilatateur tissulaire selon la revendication 2, caractérisé en ce que l'hydrogel ionique est un hydrogel non ionique saponifié.

4. Dilatateur tissulaire selon la revendication 3, caractérisé en ce que l'hydrogel non ionique est un polymère à base de méthilméthacrylate.
